# EUROPÄISCHE PATENTSCHRIFT

(11) **EP 1 774 898 B1**
(45) Veröffentlichungstag und Bekanntmachung des Hinweises auf die Patenterteilung: **25.08.2010**
(21) Anmeldenummer: 06021492.1
(22) Anmeldetag: 13.10.2006
(51) Int. Cl.: A61B 1/31

(54) **Rektoskop mit lichtabstrahlenden Elementen**
Rectoscope having light emitting elements
Rectoscope avec des éléments émettant de la lumière

(30) Priorität: 17.10.2005 DE 102005050554
(43) Veröffentlichungstag der Anmeldung: 18.04.2007
(73) Patentinhaber: Karl Storz GmbH & Co. KG, 78532 Tuttlingen (DE)
(72) Erfinder: Leroy, Joel Jules Louis Prof., 62160 Bully les Mines (FR); Mutter, Didier Raoul Daniel, 67550 Vendenheim (FR); Marescaux, Jacques Francois Bernard, 67310 Scharrachbergheim (FR); Vix, Michel Joseph Emile, 67207 Niederhausbergen (FR)
(74) Vertreter: Weller, Wolfgang

(56) Entgegenhaltungen:
- GB-A- 1 134 972
- US-A- 3 146 775
- US-A- 3 261 349
- US-A- 4 306 546
- US-A1- 2003 100 819

## Beschreibung

Die Erfindung betrifft ein Rektoskop, mit einem ein Außenrohr und ein Innenrohr aufweisenden Tubus und einem davon abstehenden Griff, wobei am distalen Ende des Tubus mehrere umgänglich verteilt angeordnete lichtabstrahlende Elemente angeordnet sind.

Ein solches Rektoskop ist aus der US 3,261,349 bekannt.

Der Tubus in Form eines beidseitig offenen Rohres weist beispielsweise eine Länge von 20 cm und einen Durchmesser von etwa 40 mm auf. Für eine transanale Rektumchirurgie wird der Tubus in das Rektum eingeführt, und durch den Tubus können Instrumente zur Durchführung von operativen Eingriffen ein- oder hindurchgeführt werden.

Rektoskope werden auch für eine so genannte End-zu-End-Anastomose eingesetzt. Bei dieser Operation wird ein Teilstück des z.B. tumorbefallenen Darms entfernt, und die zwei daraus resultierenden Enden des Darmes werden mit Hilfe eines so genannten Staplers wieder miteinander verbunden. Bei dieser Operation werden über einen Zugang im Bauchraum weitere Operationsinstrumente eingebracht. Dies sind zum einen Optiken zum Ausleuchten und Beobachten des Operationsgebietes, sowie Zangen und Scheren zum Entfernen des defekten Darmabschnittes. Des Weiteren wird auf laparoskopischem Weg eine Gegenandruckplatte eingebracht. Diese Gegenandruckplatte ist erforderlich, damit der Stapler die beiden Enden des Darms miteinander verbinden kann.

Werden Hämorrhoiden behandelt, wird durch den Tubus ein so genannter Hämorrhoiden-Ligator-Circular-Stapler hindurchgeführt, über den Klammern gesetzt werden können, um beispielsweise innenliegende Hämorrhoiden abzuklemmen.

Die Beleuchtung des Operationsgebietes kann über laparoskopisch über den Bauchraum eingeführte Optiken erfolgen.

Aus der US 3,146,775 ist ein gynäkologisches Untersuchungsinstrument bekannt, das ein Bündel aufweist, das aus lichtübertragenen Fasern besteht, die zum Beleuchten einer Untersuchungsstelle dienen. Das Bündel überträgt das Licht von einer Lichtquelle nahe einem proximalen Ende zu einer Stelle nahe einem distalen Ende des Untersuchungsinstruments. Im Bereich des distalen Ende fächern sich die Fasern des Bündels nach beiden Seiten auf und folgen spiralig der Biegung der Seitenwand, bis am distalen ein Durchblick vollständig von Fasern umgeben ist.

Die US 4,306,546 offenbart ein Endoskop, mit einem Rohr und einem mit dem Rohr verbindbaren Kopfteil. Das Rohr besteht aus einem Außenrohr und einem Innenrohr. In dem Kopfteil sind lichtübertragende Fasern angeordnet, die sich bis zu dessen distalem Ende erstrecken. Anliegend an dem distalen Ende der lichtübertragenden Fasern ist eine Lichteintrittsfläche des Außenrohrs angeordnet, so dass das Licht von den lichtübertragenden Fasern über das Außenrohr an eine Lichtaustrittsfläche des Außenrohrs übertragen wird, die an einem distalen Ende des Außenrohrs angeordnet wird. Ferner ist an dem distalen Ende des Außenrohrs eine Abstandshülse angeordnet, die die Lichtaustrittsfläche des Außenrohrs im gewissen Abstand von der zu untersuchenden Stelle am Körper des Patienten hält.

Aus der GB 1,314,972 ist ein diagnostisches Instrument bekannt, das ebenfalls einen Kopfteil und ein Rohr, das mit dem Kopfteil verbindbar ist, aufweist. In dem Kopfteil sind lichtübertragende Fasern umfänglich gleichmäßig verteilt angeordnet, die sich bis an ein distales Ende des Kopfteils erstrecken. Das Licht wird von den lichtübertragenden Fasern über das Rohr, das aus einem lichtübertragenden Material hergestellt ist, an das distale Ende des Instruments geleitet, wodurch eine Untersuchungsstelle beleuchtet wird.

Aus der US 2003/0100819 A1 ist ein Otokop bekannt, bei dem optische Fasern oder LEDs als lichtübertragende bzw. lichtabstrahlende Elemente verwendet werden.

Aus der eingangs genannten US 3,261,349 ist ein Rektoskop bekannt, das einen Tubus und einen davon abstehenden Griff aufweist. Der Tubus besteht aus einem Außenrohr und einem Innenrohr. Zwischen den beiden Rohren sind lichtübertragende Fasern angeordnet, die sich von proximal nach distal erstrecken. Die distalen Enden der lichtübertragenden Fasern sind am distalen Ende des Tubus umfänglich verteilt angeordnet und beleuchten eine Untersuchungs- bzw. Operationsstelle mit dem Licht, das von dem proximalen Ende bis zu dem distalen Ende des Rektoskops durch die lichtübertragenden Fasern geleitet wird.

Es ist Aufgabe der Erfindung zum einen die Montage eines Rektoskops zu erleichtern und zum anderen, den Schutz der lichtabstrahlenden Elemente zu gewährleisten.

Erfindungsgemäß wird die Aufgabe dadurch gelöst, dass die lichtabstrahlenden Elemente in einem am distalen Ende des Rektoskops vorhandenen Ringkörper aufgenommen sind, und dass die lichtabstrahlenden Elemente entweder die distalen Enden von Lichtleitern sind, die als Stränge ausgebildet sind, wobei die einzelnen Stränge umfänglich verteilt in dem distalseitigen Ringkörper aufgenommen sind, oder als Leuchtdioden ausgebildet sind, die umfänglich verteilt im Ringkörper eingesetzt sind.

Das Aufnehmen der lichtabstrahlenden Elemente in dem Ringkörper hat den Vorteil, dass sie in den Ringkörper eingebettet werden können.

Durch Ausbilden der lichtabstrahlenden Elemente als distale Enden von Lichtleitern wird erzielt, dass über solche Lichtleiter Licht mit hoher Lichtausbeute punktuell an das distale Ende des Tubus geführt werden kann. Die einzelnen Lichtleiter sind zu Strängen gebündelt.

Die Ausbildung der Lichtleiter als mehrere Stränge hat den Vorteil, dass die Stränge gezielt zu den umfänglich verteilt angeordneten lichtstrahlenden Stellen geführt werden können, je nach gewünschter Anordnung.

Die Maßnahme, dass die distalen Endbereiche der einzelnen Stränge in dem endseitigen Ringkörper aufgenommen sind, hat den erheblichen Vorteil, dass die lichtabstrahlenden Elemente in dem Ring fixiert und geschützt aufgenommen sind. Dies erleichtert auch die Montage. So können bspw. in dem Ringkörper, entsprechend der Anordnung, mehrere Bohrungen eingebracht werden, in die die Enden der Lichtleiter eingesteckt werden.

Die lichtleitabstrahlenden Elemente können ebenfalls als Lichtdioden ausgebildet werden. Dies hat den Vorteil, dass in dem proximalseitigen Ring fertigungstechnisch einfach Leuchtdioden eingesetzt werden können, die dann als Kompletteinheit angesetzt werden können. Es muss dann nur noch für die elektrische Energieversorgung gesorgt werden.

Die am distalen Ende des Tubus umfänglich verteilt angeordneten lichtstrahlenden Elemente haben den Vorteil, dass eine Lichtquelle zur Verfügung steht, die distalseitig vom Tubus abstrahlt.

Dies erleichtert dem Operateur die Handhabung des Rektoskopes erheblich.

Schon beim Einbringen des Tubus in das Rektum durch den gespreizten Anus ist ein lichtabstrahlendes distales Ende hilfreich.

Zum Einführen des Tubus wird üblicherweise zunächst ein konischer Spreizkörper in den Anus eingeschoben, um den Schließmuskel zu spreizen. Über diesen im Anus steckenden Spreizkörper wird dann der Tubus in das Rektum eingeschoben. Dabei entspricht der Außendurchmesser des im Anus steckenden Spreizkörpers dem lichten Innendurchmesser des Tubus. Die lichtabstrahlenden Elemente erleichtern das korrekte Ansetzen des distalen Endes des Tubus an den Spreizkörper, da der Operateur diese Stelle besser einsehen kann.

Nach dem Abziehen des Spreizkörpers wird der Tubus über einen Obturator verschlossen und mehrere Zentimeter weiter in das Rektum eingeschoben.

In das Rektoskop wird, nachdem der Obturator aus dem Rektoskop entfernt wurde, der so genannte Stapler eingeführt. Der Stapler ragt dabei über ein gewisses, dem Operateur bekanntes Maß, beispielsweise zwei Zentimeter, über das distale Ende des Rektoskopes hinaus. Aufgrund der erfindungsgemäßen Beleuchtung am distalen Ende des Rektoskopes kann man mit Hilfe der Optik, die laparoskopisch über den Bauchraum zum Operationsgebiet gebracht wurde, die Position des Staplers genau bestimmen. Da, wie zuvor erwähnt, bekannt ist, wie weit das distale Ende des Staplers vom distalen Ende des Rektoskopes vorsteht, kann diese Position relativ exakt bestimmt werden. Die distale Beleuchtung am Rektoskop verhilft zudem zu einer besseren Ausleuchtung des Operationsgebietes.

In einer weiteren Ausgestaltung der Erfindung sind die Lichtleiter in der Wand des Tubus von proximal zum distalen Ende des Tubus geführt.

Diese Maßnahme hat den Vorteil, dass die sehr schlank bauenden Bauteile in die Wand integriert werden und weder die Außenseite des Tubus, die direkt mit dem Rektum in Verbindung steht, noch die Innenseite, die zum Durchführen der zahlreichen weiteren Operationsinstrumente frei zur Verfügung steht, beeinträchtigt werden.

In einer weiteren Ausgestaltung der Erfindung sind die Lichtleiter durch den Griff zu einem endseitigen Lichtleiteranschluss geführt.

Diese Maßnahme hat den Vorteil, dass die_ Lichtleiter raumsparend zu dem Lichtleiteranschluss geführt werden können, über den sie von einer äußeren Lichtquelle mit Licht versorgt werden.

In einer weiteren Ausgestaltung der Erfindung weist der Tubus ein Außenrohr und ein Innenrohr auf, zwischen denen die Lichtleiter geführt sind.

Diese Maßnahme hat den Vorteil, dass die Lichtleiter geschützt zwischen diesen beiden Rohren aufgenommen sind.

In einer weiteren Ausgestaltung der Erfindung sind die Stränge im Griff als Bündel aufgenommen.

Diese Maßnahme hat den Vorteil, dass eine kompakte Führung der Stränge als Bündel durch den Griff hindurch möglich ist.

In einer weiteren Ausgestaltung der Erfindung sind die Stränge über eine gewisse Länge des Tubus von proximal nach distal als Bündel geführt und sind im distalen Endbereich des Tubus zu den einzelnen umfänglich verteilten Strängen aufgespleißt.

Diese Maßnahme hat den erheblichen Vorteil, dass das Bündel, umfänglich um den Tubus gesehen, nur einen geringen Platz belegt, so dass in diesem Bereich die Möglichkeit besteht, seitliche Öffnungen zum Ein- oder Durchführen von weiteren Instrumenten im Tubus vorzusehen.

Erst im distalen Endbereich spleißt das Bündel zu den umfänglich verteilten Lichtabstrahlstellen auf.

In einer weiteren Ausgestaltung der Erfindung sind die Lichtleiter an der Außenseite des Innenrohrs geführt und sind durch Halteelemente dort gehalten.

Dies erleichtert erheblich die Montage sowie die Reinigung und trägt zu einer dauerhaft festen Halterung der Lichtleiter bei.

Das Innenrohr mit den an dessen Außenseiten angebrachten Lichtleitern kann als ein erstes Modulbauteil ausgebildet werden, das, nachdem das Innenrohr mit den Lichtleitern bestückt wurde und gegebenenfalls auch die Leuchtstärke überprüft wurde, in das Außenrohr eingeschoben werden kann, wobei dann nur noch das Bündel durch den Griff hindurchgefädelt werden muss.

Dazu ist in einer weiteren Ausgestaltung vorgesehen, dass Stromkabel zur Energieversorgung der Leuchtdioden in der Wand des Tubus von proximal nach distal geführt sind.

Hier kann im Prinzip wieder wie zuvor bei den Lichtleitern erwähnt verfahren werden, also eine Bündelung und elegante Führung durch den Griff hindurch durchgeführt werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: eine Seitenansicht eines erfindungsgemäßen Rektoskopes;
- Fig. 2: eine Explosionsdarstellung des Rektoskopes mit aus dem Außenrohr abgezogenem Innenrohr mit den Lichtleitern;
- Fig. 3: eine Draufsicht auf das Innenrohr von Fig. 2;
- Fig. 4: eine Unteransicht des Innenrohrs, wobei das Außenrohr in dessen Bereich weggelassen ist, sowie am rechten Ende den proximalen Endabschnitt des Außenrohrs mit davon abstehendem Griff;
- Fig. 5: einen Schnitt im Bereich des in der Darstellung von Fig. 4 oberen linken Endes zur Erläuterung der Konstruktion des Ringkörpers und eines darin befestigten Lichtleiters; und
- Fig. 6: eine teilweise geschnittene abschnittsweise Darstellung des Rektoskops von Fig. 1, wobei gerade ein Obturator zum Verschließen des Tubus eingeschoben wird.

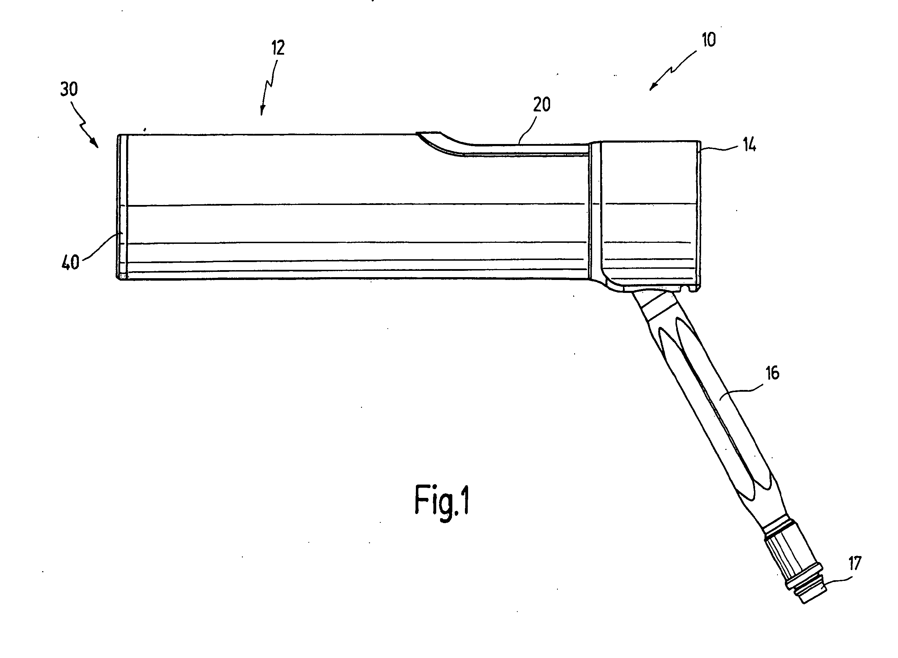

Ein in den Figuren 1 bis 5 dargestelltes Rektoskop ist in seiner Gesamtheit mit der Bezugsziffer 10 bezeichnet.

Wie insbesondere aus Fig. 1 ersichtlich, weist das Rektoskop 10 einen an beiden Enden offenen Tubus 12 auf, von dessen proximalem Ende 14 sich seitlich, schräg abstehend ein Griff 16 weg erstreckt. Der Tubus 12 ist geradlinig dargestellt, er kann auch gekrümmt verlaufen.

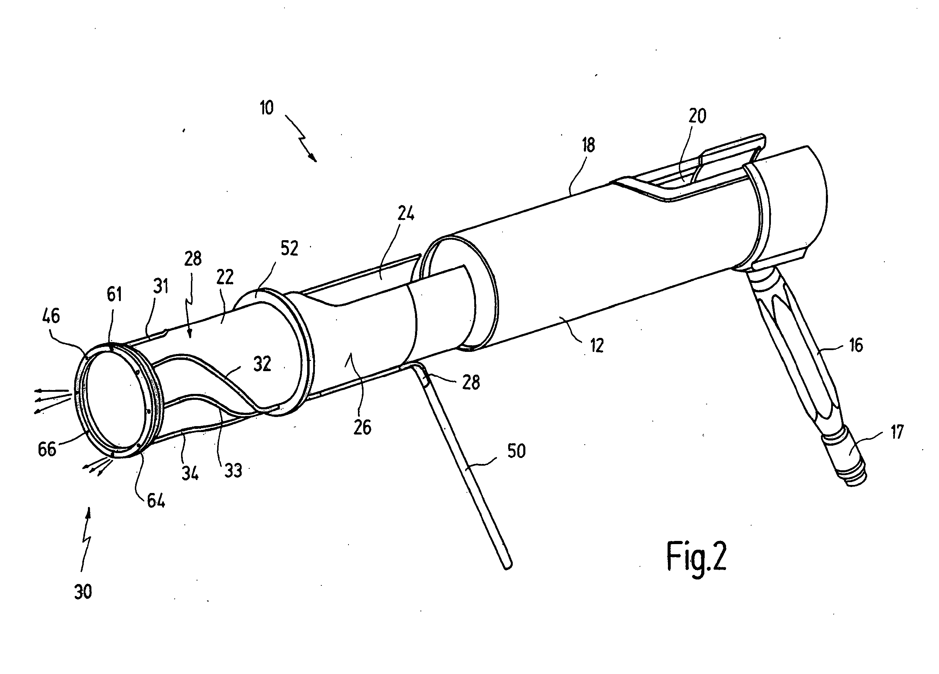

Aus der Explosionsdarstellung von Fig. 2 ist zu erkennen, dass der Tubus 12 aus einem Außenrohr 18 und einem Innenrohr 22 zusammengesetzt ist.

Das Außenrohr 18 weist an seiner Außenseite den zuvor erwähnten abstehenden Griff 16 auf.

Die Länge des Tubus 12 im dargestellten Ausführungsbeispiel beträgt etwa 16 cm, der lichte Innendurchmesser etwa 35 mm.

Gegenüberliegend zum Griff 16 ist im Außenrohr 18 ein Schlitz 20 ausgespart.

Das Innenrohr 22 weist etwa dieselbe Länge auf, wobei dessen Durchmesser jedoch etwas geringer ist, nämlich um 2 bis 3 mm, und im Innenrohr 22 ist ebenfalls ein entsprechender Schlitz 24 vorgesehen.

An der Außenseite 26 des Innenrohrs 22 sind Lichtleiter 28 angebracht.

Die Lichtleiter 28 bestehen aus acht Strängen 31 bis 38.

Jeder Strang besteht aus einer äußeren Umhüllung, sei es aus Metall oder aus Kunststoff, in der zahlreiche einzelne Glasfasern als eigene lichtleitende Körper aufgenommen sind.

Am distalen Ende 30 des Innenrohrs 22, das auch zugleich das distale Ende des Tubus 12 darstellt, ist ein Ringkörper 40 am Innenrohr 22 angeordnet.

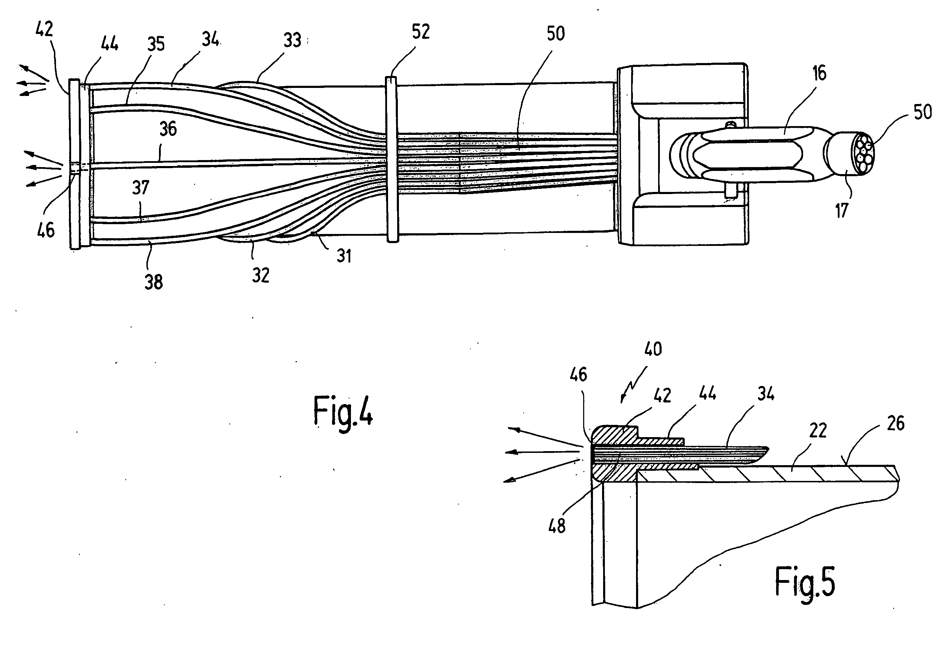

Der Ringkörper 40 ist aus einem Endring 42 zusammengesetzt, von dem sich nach proximal über einige Millimeter ein Ringflansch 44 fort erstreckt (siehe insbesondere Fig. 5).

Der Außendurchmesser des Endringes 42 entspricht etwa dem Außendurchmesser des Außenrohrs 18.

Der Außendurchmesser des Ringflansches 44 entspricht dem lichten Innendurchmesser des Außenrohrs 18. Die Innenseite des Ringflansches 44 verläuft etwas im Abstand zur Außenseite 26 des Innenrohrs 22.

Im Endring 42 sind umfänglich gleichmäßig verteilt acht Bohrungen 46 eingebracht, wobei in diese acht Bohrungen 46 jeweils das distale Ende 48 eines der Stränge 31 bis 38 eingeschoben ist. Dabei wird das distale Ende 48 unter dem Ringflansch 44 und zwischen der Außenseite 26 des Innenrohrs 22 hindurchgeführt.

Dadurch entsteht das aus den Figuren 2, 3 und 4 ersichtliche Verteilungsmuster der Stränge 31 bis 38.

Wie insbesondere aus Fig. 4 zu entnehmen ist, werden die acht Stränge 31 bis 38 zu einem Bündel 50 zusammengeführt, wobei das Bündel 50 durch den Innenraum des Griffes 16 hindurchgeführt ist, bis zu einem an dessen Außenseite angeordneten Lichtleiteranschluss 17.

In der Explosionsdarstellung von Fig. 2 ist das Bündel 50 abgewinkelt dargestellt, um darzustellen, wie es im montierten Zustand im Griff 16 aufgenommen ist.

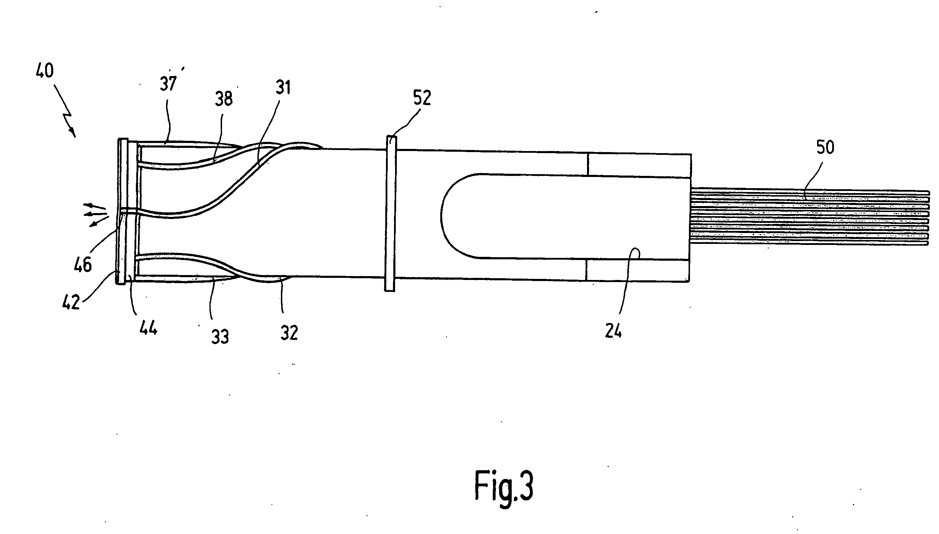

Zunächst ist die Ausrichtung des Bündels 50 wie in Fig. 3 dargestellt, damit es in das Außenrohr 18 eingeschoben werden kann.

Im Außenrohr 18 ist im Bereich des Griffes 16 eine Öffnung vorhanden, über die das Bündel 50 in den Griff 16 eingefädelt werden kann. Dies wird noch zusätzlich bei der Montage durch die beiden Schlitze 20 und 24 erleichtert.

Im endmontierten Zustand ist also das Bündel 50 aus den acht Strängen 31 bis 38 vom Griff 16 kommend über eine gewisse Längserstreckung des Innenrohrs 22 als Bündel 50 weitergeführt und wird durch ein ringförmiges Halteelement 52 zusätzlich gehalten. Anschließend spleißt das Bündel 50 auf, so dass dann die acht Stränge 31 bis 38 zu den entsprechenden acht Bohrungen 46 im Endring 42 geführt werden.

Ist im endmontierten Zustand der Griff 16 mit einer Lichtquelle verbunden, wird das Licht über die Lichtleiter 28 durch den Griff bis zum distalen Ende 30 geführt.

Dort wird das Licht an den acht distalen Enden 48 punktuell abgestrahlt.

Diese acht Abstrahlstellen bilden umfänglich verteilt angeordnete, lichtabstrahlende Elemente; hier sind nur die Elemente 61, 64 und 66 in Fig. 2 bezeichnet.

Der in den Figuren 1 bis 4 beschriebene Aufbau stellt quasi den Grundkörper eines Rektoskopes 10 dar.

Bei der Handhabung können dann durch den Hohlraum des Tubus 12 hindurch Manipulationen durchgeführt werden, beispielsweise entsprechende Instrumente hindurchgeschoben werden, um an der Innenseite des Enddarmes chirurgische Eingriffe vornehmen zu können.

Dies können beispielsweise Eingriffe an den Hämorrhoiden sein, oder auch ein Abtrennen eines tumorbefallenen Abschnittes des Enddarmes.

Die beiden verbleibenden Endstücke müssen wieder zusammengefügt werden durch eine so genannte End-zu-End-Anastomose. In diesem Fall wird in den Tubus ein so genannter Stapler eingeführt. Alle weiteren notwendigen Instrumente werden durch den Bauchraum zum Operationsgebiet gebracht.

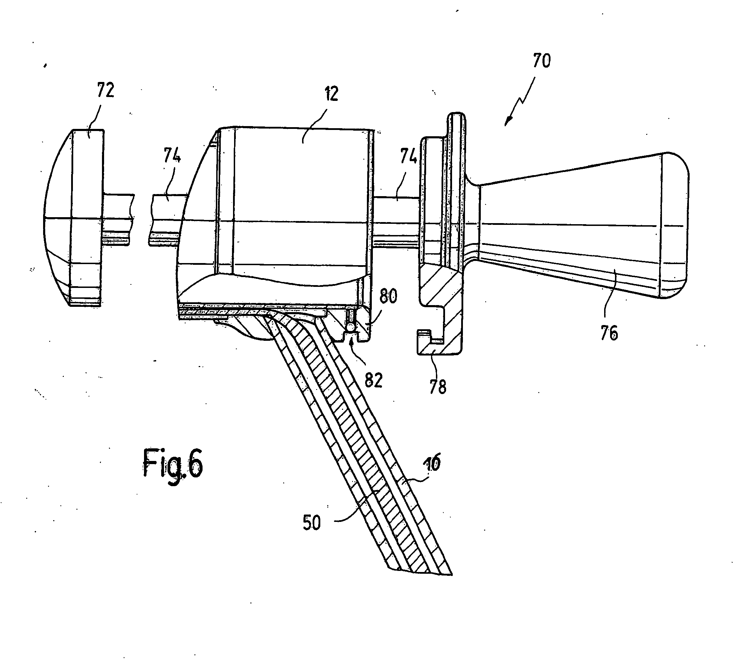

In Fig. 6 ist dargestellt, wie gerade ein so genannter Obturator 70 in den Tubus 12 von proximal eingeführt wird.

Der Obturator 70 weist distalseitig einen Stopfen 72 auf, um das distale Ende des offenen Tubus 12 zu verschließen. Über eine Stange 74 ist der Stopfen mit einer Handhabe 76 verbunden. Diese weist einen Rasthaken 78 auf, der mit einer entsprechenden Kugelraste 80 am proximalen Endbereich verrastet werden kann. Eine federbelastete Kugel 82 sorgt für die Haltekraft.

Ist der Obturator 70 so angesetzt und verrastet, ist der Tubus 12 an beiden Enden verschlossen.

Die Handhabung des Rektoskopes 10 ist beispielsweise wie folgt:

In den Anus eines Patienten wird zunächst ein sich distalseitig konisch verjüngender Spreizkörper eingeführt, um den Anus aufzuweiten. Der Außendurchmesser des Spreizkörpers entspricht etwa dem lichten Innendurchmesser des Tubus 12.

Anschließend wird der Tubus 12 über den im Anus steckenden Spreizkörper geschoben und dabei etwas in das Rektum eingeführt. Nach dem Überschieben des Tubus 12 über den Spreizkörper wird der Spreizkörper durch den Tubus 12 nach proximal abgezogen.

Nunmehr wird der Obturator 70 eingesetzt und verrastet, so dass der Tubus 12 beidseitig dicht verschlossen ist.

In diesem Zustand wird nun der Tubus 12 so weit in das Rektum eingetrieben, wie dies gewünscht ist.

Anschließend wird der Obturator 70 abgenommen, und es können dann die gewünschten Instrumente, z.B. ein Stapler, durch den Tubus 12 hindurchgeführt werden. Die distale Beleuchtung am Rektoskop erleichtert die Positionsbestimmung des Staplers und dient außerdem zur besseren Ausleuchtung des Operationsgebietes.

Bei Instrumenten mit elektrisch betriebenen Werkzeugen, beispielsweise Schneiden oder Nähvorrichtungen, müssen diese Instrumente eine elektrische Versorgung durch Kabel aufweisen.

Da Stapler üblicherweise gekrümmt ausgebildet sind, kann der proximale Endbereich über die Schlitze 20, 24 herausragen.

Durch die glatte Linienführung des Tubus, also keine Behinderung der Innenseite des Innenrohrs 22 durch irgendwelche Bauelemente, können die Instrumente zur Außenseite hin abdichtend angesetzt werden, wobei dies zusätzlich durch die Geometrie des Ringkörpers 40 begünstigt wird, der als Anlagekörper oder Anlageflansch für solche Werkzeuge am distalen Ende dienen kann.

Nach Durchführung der chirurgischen Eingriffe wird das Rektoskop 10 vom Rektum abgezogen. Je nach Ausgestaltung der Verbindung zwischen Innenrohr 22 und Außenrohr 18 können die Instrumente zu Reinigungszwecken voneinander abgenommen sowie gereinigt und sterilisiert werden.

Es ist auch möglich, wenn diese aus hochwertigen Materialien, beispielsweise Edelstahl, ausgebildet sind, diese so dichtend miteinander zu verbinden, beispielsweise durch Verlöten, dass sie zum Reinigen nicht auseinandergenommen werden müssen.

Es ist auch möglich, solche Rektoskope zum Einmalgebrauch auszugestalten, so dass dann kostengünstige Kunststoffmaterialien eingesetzt werden können.

Es sind auch Kombinationen möglich. So können beispielsweise Außenrohr 18 und Griff 16 aus Stahl bestehen, während das Innenrohr 22 samt daran montierter Lichtleiter 22 aus Kunststoffmaterialien hergestellt ist.

Zuvor wurde beschrieben, dass die Lichtleiter 28 als lichtleitende und lichtabstrahlende Elemente Glasfasern darstellen.

Es ist auch möglich, in die im Ringkörper 40 vorhandenen Bohrungen 46 Leuchtdioden einzusetzen, die dann über entsprechende Stromkabel mit Strom versorgt werden. Diese Stromkabel können dann gleichermaßen wie die Stränge 31 bis 38 geführt, gebündelt und zu einem endseitigen Stromanschluss am Griff 16 geführt werden.

## Patentansprüche

1. Rektoskop, mit einem ein Außenrohr (18) und ein Innenrohr (22) aufweisenden Tubus (12) und einem davon abstehenden Griff (16), wobei am distalen Ende (30) des Tubus (12) mehrere umfänglich verteilt angeordnete, lichtabstrahlende Elemente (61, 64, 66) angeordnet sind, **dadurch gekennzeichnet, dass** die lichtabstrahlenden Elemente in einem am distalen Ende (30) des Rektoskops (10) vorhandenen Ringkörper (40) aufgenommen sind, und dass die lichtabstrahlenden Elemente (61, 64, 66) entweder die distalen Enden (48) von Lichtleitern (28) sind, die als Stränge (31-38) ausgebildet sind, wobei die einzelnen Stränge (31-38) umfänglich verteilt in dem distalseitigen Ringkörper (40) aufgenommen sind oder als Leuchtdioden ausgebildet sind, die umfänglich verteilt im Ringkörper (40) eingesetzt sind.

2. Rektoskop nach Anspruch 1, **dadurch gekennzeichnet, dass** die Lichtleiter oder die Stromkabel zur Energieversorgung der Leuchtdioden zwischen dem Außenrohr (18) und dem Innenrohr (22) von proximal nach distal geführt sind.

3. Rektoskop nach Anspruch 1 oder 2, **dadurch gekennzeichnet, dass** die Lichtleiter (28) durch den Griff (16) zu einem endseitigen Lichtleiteranschluss (17) geführt sind.

4. Rektoskop nach Anspruch 3, **dadurch gekennzeichnet, dass** die Stränge (31-38) im Griff (16) als Bündel (50) aufgenommen sind.

5. Rektoskop nach Anspruch 4, **dadurch gekennzeichnet, dass** die Stränge (31-38) über eine gewisse Länge des Tubus (12) von proximal nach distal als Bündel (50) geführt sind, das im distalen Endbereich (30) des Tubus (12) zu den einzelnen, umfänglich verteilten Strängen (31-38) aufspleißt.

6. Rektoskop nach einem der Ansprüche 1 bis 5, **dadurch gekennzeichnet, dass** die Lichtleiter (28) an der Außenseite (26) des Innenrohrs geführt sind und durch zumindest ein Halteelement (52) dort gehalten sind.

## Claims

1. Rectoscope comprising a tube (12) having an outer pipe (18) and an inner pipe (22) and a handle (16) protruding therefrom, wherein, at a distal end (30) of the tube (12) a number of light-emitting elements (61, 64, 66) are arranged in a circumferentially distributed fashion, **characterized in that** the light-emitting elements are accommodated in an annular body (40) present at the distal end (30) of the rectoscope (10) and **in that** the light-emitting elements (61, 64, 66) are the distal ends (48) of light guides (28) which are designed as strands (31-38), wherein the individual strands (31-38) are accommodated in the distal annular body (40) circumferentially distributed or the light-emitting elements (61, 64, 66) are designed as light-emitting diodes which are inserted into the annular body (40) in an circumferentially distributed manner.

2. Rectoscope of claim 1, **characterized in that** the light guides or the power cable for supplying the light-emitting diodes with energy are guided between the outer pipe (18) and the inner pipe (22) from proximal to distal.

3. Rectoscope of claim 1 or 2, **characterized in that** the light guides (28) are guided through the handle (16) to a terminal light guide connection (17).

4. Rectoscope of claim 3, **characterized in that** the strands (31-38) are accommodated in the handle (16) as a bundle (50).

5. Rectoscope of claim 4, **characterized in that** the strands (31-38) are guided from proximal to distal over a certain length of the tube (12) as a bundle (50) which fans out in the distal end region (30) of the tube (12) to form the individual, circumferentially distributed strands (31-38).

6. Rectoscope of anyone of claims 1 through 5, **characterized in that** the light guides (28) are guided on the outside (26) of the inner pipe (22) and are held there by at least one holding element (52).

## Revendications

1. Rectoscope avec un tube (12) présentant un tube extérieur (18) et un tube intérieur (22), et une poignée (16) en saillie par rapport à celui-ci, plusieurs éléments émettant de la lumière (61, 64, 66) étant disposés à l'extrémité distale (30) du tube (12) et répartis périphériquement, **caractérisé en ce que** les éléments émettant de la lumière sont logés dans un corps annulaire (40) présent à l'extrémité distale (30) du rectoscope (10), et que les éléments émettant de la lumière (61, 64, 66) sont soit les extrémités distales (48) de guides de lumière (28) qui sont réalisés sous forme de lignes (31-38), les différentes lignes (31-38) étant réparties périphériquement dans le corps annulaire (40) côté distal, soit réalisés sous forme de diodes électroluminescentes qui sont réparties périphériquement dans le corps annulaire (40).

2. Rectoscope selon la revendication 1, **caractérisé en ce que** les guides de lumière ou les câbles électriques pour l'alimentation en énergie des diodes électroluminescentes sont guidés de proximal à distal entre le tube extérieur (18) et le tube intérieur (22).

3. Rectoscope selon la revendication 1 ou 2, **caractérisé en ce que** les guides de lumière (28) sont guidés à travers la poignée (16) vers une connexion de guide de lumière (17) terminale.

4. Rectoscope selon la revendication 3, **caractérisé en ce que** les lignes (31-38) sont logées dans la poignée (16) sous forme de faisceau (50).

5. Rectoscope selon la revendication 4, **caractérisé en ce que** les lignes (31-38) sont guidées de proximal à distal sur une certaine longueur du tube (12) sous forme de faisceau (50) qui se sépare dans la zone d'extrémité distale (30) du tube (12) en lignes individuelles (31-38) réparties périphériquement.

6. Rectoscope selon une des revendications 1 à 5, **caractérisé en ce que** les guides de lumière (28) sont guidés sur le côté extérieur (26) du tube intérieur et y sont maintenus par au moins un élément de maintien (52).
